# EUROPEAN PATENT SPECIFICATION

(11) **EP 3 160 328 B1**
(45) Date of publication and mention of the grant of the patent: **06.06.2018**
(21) Application number: 15729201.2
(22) Date of filing: 19.06.2015
(51) Int. Cl.: A61B 5/00, G06F 19/00, G08B 21/04

(54) **DEVICE, SYSTEM AND COMPUTER PROGRAM FOR DETECTING A HEALTH CONDITION OF A SUBJECT**
VORRICHTUNG, SYSTEM UND COMPUTERPROGRAMM ZUR ERKENNUNG EINES GESUNDHEITSZUSTANDS EINER PERSON
DISPOSITIF, SYSTÈME ET PROGRAMME D'ORDINATEUR POUR DÉTECTER UN ÉTAT DE SANTÉ D'UN SUJET

(30) Priority: 30.06.2014 EP 14175028
(43) Date of publication of application: 03.05.2017
(73) Proprietor: Koninklijke Philips N.V., 5656 AE Eindhoven (NL)
(72) Inventor: HEINRICH, Adrienne, NL-5656 AE Eindhoven (NL); TATOUSEK, Jan, NL-5656 AE Eindhoven (NL)
(74) Representative: Zhu, Di
(86) International application number: PCT/EP2015/063784
(87) International publication number: WO 2016/000979

(56) References cited:
- US-A1- 2011 118 573
- US-A1- 2011 245 629
- US-A1- 2013 182 107
- BIN YIN ET AL: "Detection of sensor wearing positions for accelerometry-based daily activity assessment", PROCEEDINGS OF THE SIXTH IASTED INTERNATIONAL CONFERENCE ON BIOMEDICAL ENGINEERING, INNSBRUCK, AUSTRIA, FEBRUARY 13-15, 2008, ACTA PRESS, ANAHEIM, CA, USA, 13 February 2008 (2008-02-13), pages 390-395, XP009134172, ISBN: 978-0-88986-721-5

## Description

### FIELD OF THE INVENTION

The present invention relates to a device, system and computer program for detecting a health condition of a subject. In particular, the present invention relates to reliable and early detection of worsening of chronic diseases that have already been diagnosed. It finds application in hospital as well as home healthcare monitoring, in particular during therapy and chronic disease management.

### BACKGROUND OF THE INVENTION

In healthcare management both in hospital and at home, monitoring a patient's health state is crucial for detecting a health condition of the patient with a sufficient degree of correctness and efficiency. During therapy and chronic disease management, which often has a long duration and is thus expensive, it is particularly important to detect worsening of the patient's condition as soon as possible after its onset. In this way, self-management activities and healthcare interventions can be launched in time in order to effectively and efficiently reduce severity of the episode or manage the disease. This is crucial for improving the therapy outcomes and the quality of life (QoL) of the patient as well as to reduce the overall therapy costs.

The current healthcare pathways, in particular the chronic pathways, heavily depend on the patient to initiate interventions and optimization of his therapy, based on symptoms and the overall condition he is experiencing. This healthcare activation role of the patients is not likely to weaken or diminish any time soon, despite all the attempts to introduce a more proactive approach of healthcare providers.

The current approaches are based either on objective measurements, which are not personalized or optimized for an individual patient, or on subjective input, which often signals the event too late. At the same time a lot can be improved in terms of supporting the patient in performing this role better, i.e. more reliably or more consistently. The present situation is that the patient does not have any other source of information but his own subjective interpretation of his condition. That is largely influenced by his momentary psychosomatic state and so is the threshold to trigger healthcare intervention. As a result the different patients or even the same patient in different situations triggers the healthcare in a large variety of moments from far too early, resulting in overload of healthcare resources up to and predominantly far too late. As a consequence, this results in poor outcomes and escalation of event to conditions that are too expensive to treat. The benefit of making patients and physicians aware of changes in the health condition at an early stage is obvious in improved performance of the therapy feedback loops, with positive impact on patient QoL, therapy outcomes, meaningful use of resources and overall cost of therapy.

US 2013/0182107 A1 discloses a system for monitoring a patient comprising a video camera that captures images of a patient's location, wherein the captured images are received by a video processing device and compared with preceding images to determine motion levels. A motion alert level signal is determined from the motion levels and compared with a motion alert threshold in order to generate a motion alert signal when the motion alert level signal exceeds the motion alarm threshold. Further, the system enables receiving user input for determining at least one region of the captured images.

US 2011/0245629 A1 discloses a therapy system capable of displaying a temporal correlation between bioelectrical brain signal of a patient and patient motion data, such as a gisnal indicative of patient motion of a patient posture indicator.

### SUMMARY OF THE INVENTION

It is an object of the present invention to provide a device, system and computer program for detecting a health condition of a subject which enable user-proactive disease management with increased accuracy and reliability of the detected health condition.

In a first aspect of the present invention, a device for detecting a health condition of a subject is presented comprising a data interface for receiving sensor data of the subject and one or more disease classification parameters for characterizing one or more diseases, a user interface for receiving a user input related to a disease activity perceived by the subject (15), wherein said disease activity comprises at least the occurence of one or more symptoms, an analysis unit for extracting one or more physiological and/or behavioral features from the received sensor data, an optimizer unit for optimizing the one or more disease classification parameters based on a correlation analysis between the one or more physiological and/or behavioral features and the user input, and a detection unit for detecting a health condition of the subject by applying the one or more optimized disease classification parameters to the one or more physiological and/or behavioral features.

The sensor data may comprise data acquired using one or more sensors, in particular motion sensors, configured to monitor physiological and/or behavioral data of a patient, in particular motion vectors, motion speed and/or angle. The physiological and/or behavioral features may be body activity levels indicating the level of a patient's body activity, breathing rates, temporal patterns such as scratching, getting out of bed, walking, chest expansion, respiration rate. The disease classification parameters may comprise physiological and/or behavioral and/or biological and/or chemical and/or physical parameters suitable for characterizing a disease, in particular depending on the type of sensor data. In particular, the disease classification parameters may comprise parameters related to physiological and/or behavioral features. The health condition may be a disease state or a change of the disease state of the patient, such as a flare-up or deterioration, in particular a psychosomatic deterioration of his/her condition. In particular, the health condition may comprise worsening of a disease. The disease activity describes the activity of a disease, such as the occurrence of one or more symptoms (a physical or mental feature which is regarded as indicating a condition of the user). In particular, the user input related to the disease activity is the subjective perception of the user/patient about the activity of his own disease or about a specific symptom of the disease. Preferably, the user input comprises a level, in particular a quantitative level of disease activity perceived by the user/patient, either absolute or relative to a previous time period.

Advantageously, the present invention enables to combine the user input and the one or more physiological and/or behavioral features to perform a correlation analysis. This increases the reliability of the parameter optimization, since information acquired both from objective measurements and from the user's subjective perception are utilized. By means of a correlation analysis, which is based on one or more correlation functions, the correlation between the user input and the physiological and/or behavioral features can be analyzed with high accuracy. In particular, the user input can be used to search for information from the extracted physiological and/or behavioral features that is most relevant to the user's subjective perception about the disease activity. On one hand, this may act as a "filter" to select the most useful information for detecting health conditions, leading to increased efficiency; on the other hand, this also enables a personalized parameter optimization, leading to increased reliability.

In a preferable embodiment, the optimizer unit is configured to correlate the user input with the one or more physiological and/or behavioral features of a time different from a user input time, in particular earlier than the user input time. The user input time is understood as the time at which the user interface receives a user input. In this way, if the user/patient subjectively senses a disease activity or an episode, such as a flare-up, a deterioration or an exacerbation of a disease, later than the episode's actual onset, the present invention can look into the history of measured sensor data and correlate the user input with the previously extracted physiological and/or behavioral features in order to find information therein relevant to the user's subjectively perceived disease activity or episode. This information can be used to optimize the disease classification parameters. The optimized parameters can thus be applied in the future to detect a future disease activity or episode not later or even earlier than the patient's own subjective perception. The present invention thus overcomes the problem that most patients tend to sense a disease activity or episode significantly later than the time the disease activity or episode occurs.

In a preferable embodiment, the optimizer unit is configured to optimize the one or more disease classification parameters within a predefined time interval after the user interface has received the user input. Advantageously, the present invention enables optimization of the disease classification parameters whenever a user has performed an input to the user interface. In this way, a parameter optimization "on demand" is realized while unnecessary computing power can be avoided when no user input through the user interface has been registered. By choosing an appropriate time interval between receiving of user input and parameter optimization, the present invention is able to perform parameter optimization efficiently.

In another preferable embodiment, the optimizer unit is configured to define a combination from the one or more physiological and/or behavioral features. Advantageously, the present invention enables to focus on the relevant and important physiological and/or behavioral features typical for the subject and to combine them in a proper way, such as in a proper order and/or with a proper factoring for the individual features. The efficiency and reliability of the parameter optimization is thus further increased.

In another preferable embodiment, the optimizer unit is configured to derive at least one symptomatic characteristic from the one or more disease classification parameters, the symptomatic characteristic being configured to indicate a development and/or an activity state of the at least one disease. The symptomatic characteristic may comprise a Detected Disease State (DDS) characteristic which can be used for detecting a DDS. Preferably, the stable disease states such as high or low disease activity, remission may be detected using the at least one symptomatic characteristic. Further preferably, changes between stable states, such as increasing or decreasing disease activity, may also be detected. Advantageously, the present invention is able to optimize the disease classification parameters so that when they are applied to the physiological and/or behavioral features, the health condition of the patient can be detected more specifically by relating to a development and/or an activity state of a disease. A disease-specific detection can be realized. Further, the health condition detection can be performed more individually in a way that is adapted to the individual patient's personal health situation.

In another preferable embodiment, the symptomatic characteristic is configured to indicate a disease activity decrease and/or a remission of at least one disease. Advantageously, the present invention is able to provide not only a qualitative but also a quantitative detection of health condition of the subject. The symptomatic characteristic may preferably comprise a baseline pattern which is a pattern related to the one or more physiological and/or behavioral features. The baseline pattern is configured to indicate that the disease activity is relatively low or when the disease activity decreases or when a remission of the disease takes place. Upon detecting low disease activity, the therapy or the disease management may be adapted in time to optimize the therapeutic effect.

In another preferable embodiment, the symptomatic characteristic is configured to indicate an activity increase of at least one disease. Advantageously, the therapy or the disease management may be adapted in time upon detecting high disease activity, thereby reducing the impact of the disease on the patient. In still a further preferable embodiment, the symptomatic characteristic is configured to indicate a disease activity change between two activity states of a disease. This is advantageous since the health condition detection is not only possible for stable disease states but also variable disease states, yielding in a more flexible detection.

In another preferable embodiment, the symptomatic characteristic comprises a dynamic component variable in time. In this way, the present invention is able to detect a health condition accurately even if the physiological and/or behavioral features typical for the health condition have changed over time. This is advantageous for detecting an episode of a disease, such as a flare-up or a deterioration, even when the symptoms characterizing the episode have changed after a time period. Such changes can be accounted for by means of the dynamic component of the symptomatic characteristic so that the disease classification parameters can be optimized accordingly. For instance, if a psoriasis patient reacted on upcoming flare up by increased tossing and turning in bed and two years later scratching his main reaction became his main discriminating behavior, the present invention will recognize it and shift weights in the disease classification parameters from tossing and turning to scratching. This example is, however, not limited to the case of psoriasis, but generally applicable to other diseases as well.

In another preferable embodiment, the device according to the present invention is further configured to derive at least one average disease classification parameter from a plurality of individual disease classification parameters. Advantageously, the present invention enables utilizing relative disease classification parameters. This may be done before the parameter optimization has been performed. For instance, an initial set of disease classification parameters comprising disease classification parameters from a plurality of individual patients/users may be received through the data interface. By deriving an average disease classification parameter, a highly representative initial parameter may be determined. "Average" refers to the value and/or the level of accuracy and/or the number of quantities and/or other variables of a disease classification parameter known in the art.

In another preferable embodiment, the user interface is configured to compute a relative disease activity level based on a plurality of disease activity level inputs. In this way, random and/or unwanted fluctuations of user inputs can be averaged out so that the present invention enables to utilize the user inputs more reliably. The relative disease activity level may be based on a plurality of previous disease activity level inputs, which may preferably be specified to be from a predetermined time period.

In another preferable embodiment, the optimizer unit is configured to load the optimized one or more disease classification parameters to a storage medium. In this way, one or more data files containing the optimized disease classification parameters may be stored for future utilization with the same device or a different device. This leads to increased user flexibility and more efficient resource utilization.

In a further aspect of the present invention, a system for detecting a health condition of a subject is presented comprising a monitoring unit comprising one or more sensors for measuring and/or monitoring sensor data from the subject and a device as disclosed herein for detecting a health condition of the subject by processing the measured and/or monitored sensor data. The system according to the present invention advantageously enables both obtaining sensor data and processing the obtained sensor data to detect a health condition with high reliability and efficiency, while qualitative and quantitative user inputs are involved.

In another aspect of the present invention, a computer program for detecting a health condition of a subject is presented comprising the steps of receiving sensor data of the subject and one or more disease classification parameters for characterizing one or more diseases, receiving a user input related to a disease activity, extracting one or more physiological and/or behavioral features from the received sensor data, optimizing the one or more disease classification parameters based on a correlation analysis between the one or more physiological and/or behavioral features and the user input, and detecting a health condition of the subject by applying the one or more disease classification parameters to the one or more physiological and/or behavioral features.

In yet further aspects of the present invention, there are provided a computer program which comprises program code means for causing a computer to perform the steps of the method disclosed herein once the computer program is carried out on the computer, as well as a non-transitory computer-readable recording medium that stores therein a computer program product, which, when executed by the device, causes the method disclosed herein to be performed.

Preferable embodiments of the invention are defined in the dependent claims. It shall be understood that the claimed system, method and computer program have similar and/or identical preferable embodiments as the claimed device and as defined in the dependent claims.

### BRIEF DESCRIPTION OF THE DRAWINGS

These and other aspects of the invention will be apparent from and elucidated with reference to the embodiments described hereinafter. In the following drawings
Fig. 1 shows a schematic block diagram of a first embodiment of a device according to the present invention;
Fig. 2 shows a schematic block diagram of a second embodiment of the device according to the present invention;
Fig. 3 shows a schematic block diagram of a third embodiment of a device according to the present invention;
Fig. 4 shows a schematic block diagram of a plurality of the device in Fig. 1 in a connected mode;
Fig. 5 shows a motion speed measured during a repetitive movement; and
Fig. 6 shows a schematic block diagram of an embodiment of a system according to the present invention.

### DETAILED DESCRIPTION OF THE INVENTION

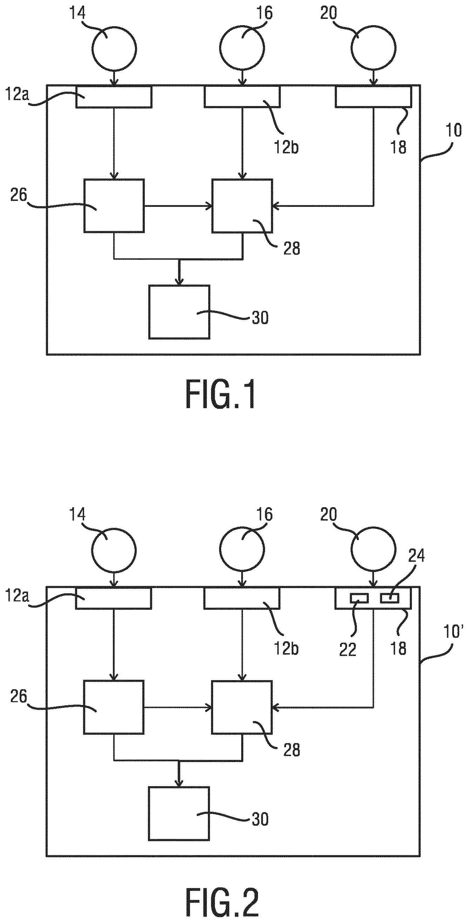

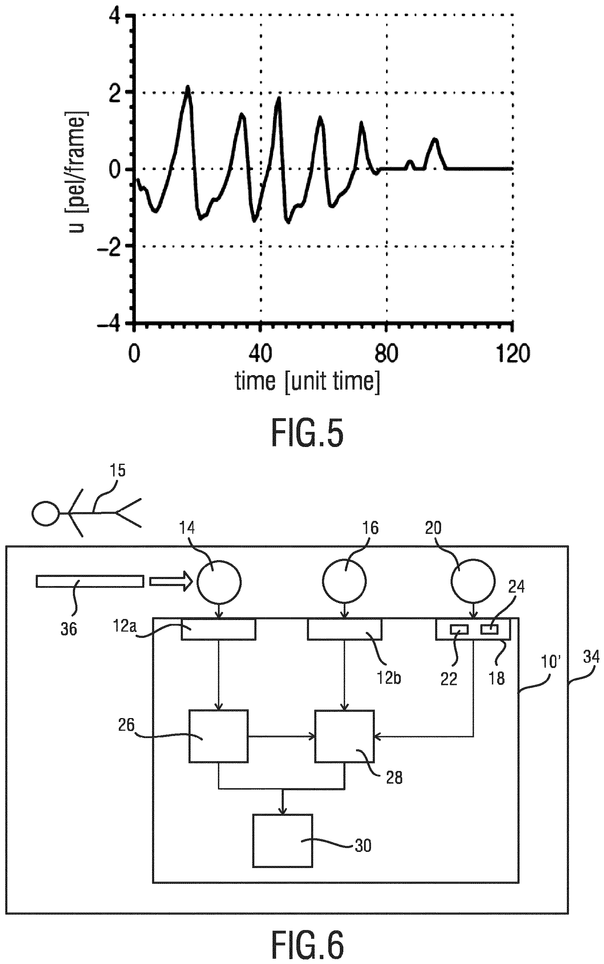

With reference to Fig. 1, a schematic block diagram of a device 10 for detecting a health condition of a subject is shown in accordance with a first embodiment. The device 10 comprises a data interface 12 for receiving sensor data 14 of the subject 15 (Fig. 6) and one or more disease classification parameters 16 for characterizing one or more diseases. The data interface 12 may comprise any type of data interface known in the art, in particular RS-232, GPIB, C.35, Current Loop, etc., without being limited to the afore-mentioned types of data interface. The data interface 12 may comprise two spatially separated interface elements 12a, b, one for receiving sensor data 14 and the other for receiving disease classification parameters 16, as is shown in Fig. 1. It is, however, understood that the data interface 12 may alternatively comprise one single interface element for receiving both the sensor data 14 and the disease classification parameters 16.

The device 10 further comprises a user interface 18 for receiving a user input 20 related to a disease activity. The user interface 18 may comprise any type of interfaces, including Graphic User Interface (GUI), command-based user interface, text-based user interface, without being limited to the afore-mentioned types. The user input 20 may comprise a text input, a voice input, a visual input, containing information related to the user's subjectively perceived disease activity or episode.

The device 10 further comprises an analysis unit 26 for extracting one or more physiological and/or behavioral features from the received sensor data 14. Preferably, the analysis unit 26 is configured to extract the one or more physiological and/or behavioral features using one or more feature extraction algorithms. The physiological and/or behavioral features may be bed movements such as tossing, turning and scratching, changing of sleeping positions, or other body motions. Alternatively, other types of physiological and/or behavioral features including, without being limited to, changes of body temperature, changes of blood pressure, aspiratory features, sounds, etc.

The device 10 further comprises an optimizer unit 28 for optimizing the one or more disease classification parameters 16 received through the data interface 12. The optimizer unit 28 optimizes the disease classification parameters 16 by correlating the user input 20 with the one or more physiological and/or behavioral features extracted from the sensor data 14 using a correlation analysis. In this way, the information contained in the user input 20 is utilized to "filter" the one or more extracted features so that information from such features most relevant to the user's subjective perception about the disease activity or an episode can be derived from the extracted features and used for optimizing the disease classification parameters 16. Preferably, the optimizer unit 28 is configured to perform the optimization of the disease classification parameters 16 within a predefined interval after the user input 20 has been received through the user interface 18.

The correlation analysis used by the device 10 normally utilizes one or more correlation functions which comprise one or more correlation coefficients. Such correlation coefficients may comprise Pearson's product-moment coefficient, rank correlation coefficients including Spearmen's rank correlation coefficients and Kendall's rank correlation coefficients, correlation matrices, without being limited to the afore-mentioned correlation coefficients. A correlation analysis is able to correlate the one or more extracted features to the user input so that relevant features may be selected and combined.

In another preferable embodiment, the optimizer unit 28 is configured to search for predictors of the user input 20 among the one or more physiological and/or behavioral features extracted from the sensor data 14, in order to perform the parameter optimization. In a preferable embodiment, searching the predictors is done by a correlation analysis, which is performed whenever the user interface 18 receives a new user input 14, preferably after a predefined time interval after the user interface 18 has received the new user input 14. The predictor is preferably a combination of several features from the one or more physiological and/or behavioral features extracted from the sensor data 14, in particular sensor data 14 received during a night, and/or previously extracted physiological and/or behavioral features and/or previously received one or more user inputs 20. In particular, such a combination may comprise one or more predictor variables of the user input 20 among the one or more physiological and/or behavioral features. This is advantageous since it takes into account that each patient has his or her own set of physiological and/or behavioral early markers of an increasing disease activity. In this way, the present invention allows to adapt the detection process to each individual patient. By focusing on the strongest markers, worsening of the patient's condition may be detected from subliminal activity at nights, several days before the patient can notice it.

Preferably, the optimizer unit 28 is configured to correlate the user input 20 with the one or more physiological and/or behavioral features of a time different from a user input time, in particular earlier than the user input time. Upon identifying information relevant to the user's subjectively perceived disease activity or episode in the data history, this information may be used to optimize the disease classification parameters 16. The optimized disease classification parameters 16 may be used for future detection of health condition, allowing an early detection, in particular a detection of health condition earlier than the patient himself can subjectively sense the health condition. This allows surgical persons to apply and/or adjust their therapy ahead of time and avoid costs resulting from late detection.

In a further preferable embodiment, the optimizer unit 28 is configured to derive a symptomatic characteristic from the one or more disease classification parameters 16, wherein the symptomatic characteristic is configured to indicate a development and/or an activity state of the at least one disease. In particular, the symptomatic characteristic is configured to indicate an activity state of the disease. The symptomatic characteristic may preferably be configured to indicate an activity decrease and/or a remission and/or an activity increase of at least one disease. In particular, the symptomatic characteristic may be a baseline pattern of the disease which is the pattern related to the one or more physiological and/or behavioral features and typical for a relatively low disease activity. In particular, the baseline pattern may be configured as a combination of values of one or more variables and/or their temporal patterns contained in the one or more physiological and/or behavioral features. Further preferably, the symptomatic characteristics may be utilized to detect a flare-up or a deterioration of one or more diseases in a subject 15. The disease classification parameter 16 optimized with the help of the symptomatic characteristics hence enable early detection of upcoming flare-ups or deterioration of the disease. Preferably, the symptomatic characteristic derived from the disease classification parameters 16 comprises at least one dynamic component which is variable in time.

The device 10 further comprises a detection unit 30 for detecting a health condition of the subject 15. The detection unit 30 detects the health condition by characterizing the at least one disease in one or more physiological and/or behavioral features using the one or more disease classification parameters 16. The disease classification parameters 16, in particular after having been optimized by the optimizer unit 28, are applied to the one or more physiological and/or behavioral features extracted from the sensor data 14. The health condition may be the presence of one or more diseases, the level of developments of the one or more diseases, the level of disease activity of one of more diseases, the risk of worsening of symptoms, the occurrence of one or more symptoms, or other kinds of health condition known in the art.

With reference to Fig. 2, a schematic block diagram of a device 10' in accordance with another embodiment is shown. The device 10' in Fig. 2 is essentially the same as the device 10 shown in Fig. 1, except that the user interface 18 comprises a disease input element 22 configured to enable a disease input and a level input element 24 configured to enable a disease activity level input of the disease. Preferably, the disease input element 22 may comprise a text input element such as a keyboard or a touch screen. In another preferable embodiment, the disease input element 22 may comprise a voice input element so that the voice of the user may be recorded and the disease contained in the voice message of the user may be extracted. One or more names of diseases may be stored and displayed by one or more buttons, so that the user may press the one or more buttons to enter the disease input. Preferably, the level input element 24 may comprise one or more buttons or knobs, so that the user may enter the disease activity level input into the device 10. Further preferably, the user interface 18 is configured to enable or cooperate with one or more disease activity quantification methods, in particular Disease Activity Score-28 (DAS28), AKA, Joint Count, etc. In another preferable embodiment, the level input element 24 comprises a voice input unit, so that a voice message of the user containing the level of disease activity perceived by the user, in particular the patient, may be recorded. In a further preferable embodiment, the disease input element 22 and the level input element 24 are configured as a single input element, preferably a voice input element, so that a voice message of the user containing both the disease input and the disease activity level input may be recorded and extracted. In another preferable embodiment, the user interface 18 enables to register the time when the user/patient performs the user input 20 and/or a part of the user input 20 such as the disease input and/or the disease activity level input.

The present invention hence enables a more accurate and efficient detection of health conditions than devices, systems and methods known from the art. By enabling a disease input and a disease activity level input, the user, in particular the patient, is assisted in providing more accurate information for the detection process. In particular, the patient is assisted in providing not only qualitative information about the disease, but also quantitative information about its level of disease activity. This is advantageous, since when a patient is suffering from a disease, it becomes difficult for him/her to maintain the mental state required to provide the correct information. Hence, the user interface according to the present invention increases the reliability of the patient's input and the overall reliability of the health condition detection when such input is utilized.

Further, using the disease activity level input also simplifies the optimization process of the disease classification parameters. Compared to merely qualitative inputs, the disease activity level input is quantitative and can be directly integrated by the optimizer unit when optimizing the disease classification parameters. This reduces the encoding efforts which would be necessary when purely qualitative input would be implemented during parameter optimization, leading to a reduced chance of errors due to misinterpreting. Hence, the disease classification parameters can be optimized more reliably while taking into account each patient's own development of a health condition.

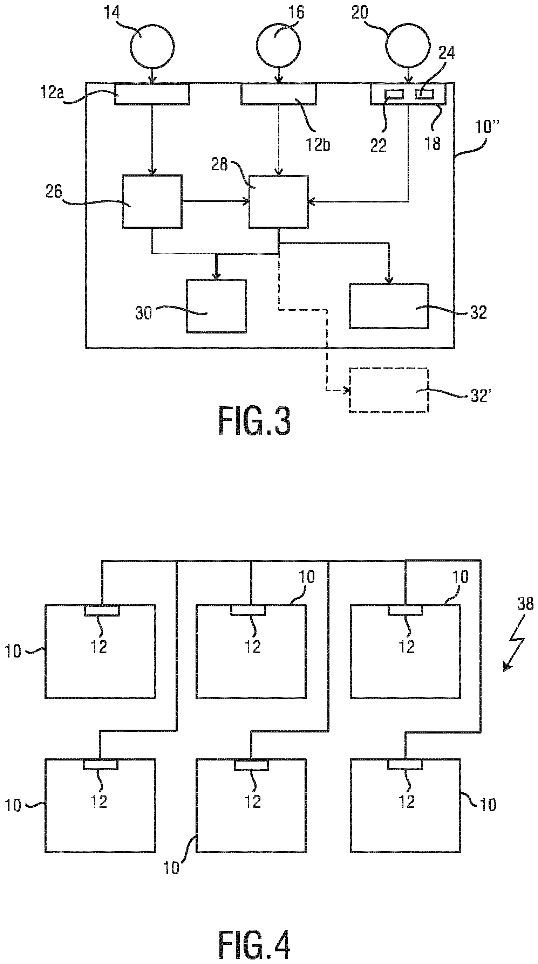

With reference to Fig. 3, a schematic block diagram of the device 10" in accordance with another embodiment is shown. The device 10" of Fig. 3 comprises essentially the same components as the device 10' of Fig. 2, except that the device 10" further comprises a storage medium 32 configured to store the one or more disease classification parameters 16 after they have been optimized by the optimizer unit 28. The storage medium 32 may alternatively be configured to be an external storage medium 32' (shown as dashed line in Fig. 3). With the help of the storage medium 32, the disease classification parameters 16 may be stored as data files, in particular each being for characterizing one specific disease and/or one specific disease profile. The storage medium 32 may be any kind of storage medium known in the art, such as flash memory, phase change memory, magnetic memory, photonic memory, etc., without being limited to the afore-mentioned types of storage media. It is understood that the device 10' of Fig. 2 may also comprise such a storage medium 32, 32'. In a preferable embodiment, the storage medium 32' is configured as a mobile memory card, in particular an SIM card that can be inserted into a database system. The database system may in particular be a universal platform supporting a rich set of data files for one or more diseases, in particular chronic diseases. Such data files may be utilized to detect a health condition with high efficiency and reliability since they are based on relevant physiological and/or behavioral features measured from the subject 15 and on the user input 20 provided by the subject 15 or another user. In a preferable embodiment, the data files contain each a set of disease classification parameters 16 according to a symptomatic characteristic. Such data files may be utilized to detect a development and/or activity state of at least one disease with high reliability. It is understood that the storage medium 32, 32' may be included into the device 10 in Fig. 1 as well.

In another preferable embodiment, the storage medium 32 is configured to enable or cooperate with a backup mechanism for creating a backup of one or more data files stored in the storage medium 32. In still a further preferable embodiment, the storage medium 32 is configured to store the user input 20 and/or the disease classification parameters 16 and/or the sensor data 14 and/or the one or more physiological and/or behavioral features. This enables the device 10, 10', 10" to look into the previously measured and/or received sensor data 14, in particular whenever a new user input 14 has been performed. Advantageously, this enables to apply a correlation analysis between the user input 14 on one hand and the one or more physiological and/or behavioral features on the other hand safely and repeatablly. This can be done either locally within the device 10, 10', 10" or externally, depending on the realization of the storage medium 32. The disease classification parameters 16 may thus be optimized according to the result of the correlation analysis.

With reference to Fig. 4, a schematic block diagram is shown in accordance with a plurality of the device 10 in Fig. 1 in a connected mode. Here, a plurality of the devices 10 shown in Fig. 1 are connected via the individual data interfaces 12. It is understood that the device 10 in Fig. 4 may also be replaced by the device 10' in Fig. 2 or the device 10" in Fig. 3. The devices 10 in Fig. 4 are thus able to exchange data with each other, in particular to exchange the user input 20 and/or the disease classification parameters 16 and/or the sensor data 14 and/or the one or more physiological and/or behavioral features. The network realized in this way may serve as a central processing system (CPS) 36. In another preferable embodiment, the central processing system 36 comprises a central communication unit to which one or more of the plurality of devices 10 are connected via the individual data interfaces 12. Preferably, other devices and/or systems may be connected to the CPS 36 via the central communication unit so that their data may also be exchanged with the one or more devices 10.

In all the embodiments disclosed herein, the one or more diseases may be Rheumatoid arthritis (RA), chronic obstructive pulmonary disease (COPD), Congestive Heart Failure (CHF), Crohn's disease, Psoriasis, and Ankylosing Spondylitis, etc., without being limited to the afore-mentioned diseases. In the case of RA, the one or more physiological and/or behavioral features may be unnatural sleeping positions and/or reduced movements during sleep and/or difficulties in moving extremities and/or difficulty in getting out of the bed and/or longer duration of getting out of the bed. Related to these physiological and/or behavioral features, disease classification parameters such as the body activity level may be computed from one or more motion vectors or one or more motions detected from the sensor data 14, in particular in video sequences. In particular, the body activity level may be a level of global full body actigraphy and/or local body part motion. In a preferable embodiment, the movement duration may be computed based on body activity level count above a threshold. In another preferable embodiment, the body activity level may be compared with that derived from "good" nights, i. e. from nights where the patient has shown a normal body activity level. Greatly reduced body activity level indicates reduced movements and/or moving difficulties.

In the case of COPD, the physiological and/or behavioral features may be restlessness and/or search for sitting position and/or breathing pattern and/or catching breath and/or cough, etc. The corresponding disease classification parameters 16 may be related to frequency analysis of motion vectors. In a preferable embodiment, the frequency analysis of motion vectors is configured so that it can return abrupt movements. In a preferable embodiment, the physiological and/or behavioural features are detected using motion analysis in combination with audio analysis. A high body activity level indicates restlessness of the subject 15.

In the case of Crohn's disease, the physiological and/or behavioral features may be cramps and motions associated with toilet visits at night. The corresponding disease classification parameters 16 may include the position coordinates of the subject 15 in order to detect change of motion area in respect of a region of interest such as the bed area. A video motion area indicates the area of motion of the patient. Whenever the patient exits the region of interest being the bed area, the monitoring unit 36 detects an Out-of-bed motion, and vice versa.

In the case of Psoriasis, the physiological and/or behavioral features may be scratching motions, while the corresponding disease classification parameters 16 may include motion speed and/or motion frequency and/or motion angle of the subject 15. Fig. 5 shows a measurement result of the motion speed (vertical axis) during a repetitive movement in bed as a function of time (horizontal axis). From the measurement result, the speed and/or the frequency of the monitored motion can be derived and analyzed to extract the physiological and/or behavioral features.

In the case of Ankylosing Spondylitis, the physiological and/or behavioral features may be motions associated with morning stiffness, while the corresponding disease classification parameters 16 may include body activity level of the subject 15. A decrease in the body activity level of the subject 15 can be programmed to indicate reduced movements.

In a preferable embodiment, the device 10, 10', 10" is configured to derive an initial set of disease classification parameters 16, in particular by collecting disease classification parameters 16 from devices and/or systems, for instance, devices and/or systems that are in use and/or already have been used. Data analysis may be applied to the collected disease classification parameters 16 in order to create an optimal set of initial disease classification parameters 16 for the individual user. In this way, the present invention does not need to additionally adapt itself to the individual user but can start directly with optimal disease classification parameters 16. This is advantageous compared to systems known in the art where one has to start with some initial set of parameters and only after some time the system adapts itself to the individual user. The present invention thus enables a more efficient detection of health condition.

In another embodiment, the device 10, 10', 10" is configured to derive a plurality of sets of initial disease classification parameters 16 and determine from the plurality of sets an optimal parameter set. Preferably, the optimal parameter set may be determined based on the user input 20 and/or the one or more physiological and/or behavioral features, in particular at the start or at an early stage of receiving and/or monitoring the one or more physiological and/or behavioral features. Preferably, an initial/optimal set of disease classification parameters 16 may be derived for men, while a different initial/optimal set of disease classification parameters 16 may be derived for women. In still a further preferable embodiment, the data interface 12 comprises an output interface element for sending the sensor data 14 and/or the disease classification parameters 16 received by the data interface 12 to a CPS and/or the database system, in particular the universal platform. In this way, the device 10, 10', 10" is connected to the CPS and/or the database system. Preferably, the connectivity between the device 10, 10', 10" and to the central processing system and/or the database system is active during a plurality of data exchange sessions and inactive outside the data exchange sessions.

In all embodiments disclosed herein, the device 10, 10', 10" is further configured to signal the user/patient about the detected health condition. In this way, the patient can be made aware of a deterioration of one or more diseases or a flare-up of the disease.

With reference to Fig. 6, a schematic block diagram of the system 34 for detecting a health condition of a subject in accordance with an embodiment is shown. The system 34 comprises the device 10' as disclosed herein with reference to Fig. 2 and a monitoring unit 36 comprising one or more sensors for measuring and/or monitoring sensor data from the subject 10. It is understood by a skilled person that the system 34 may comprise the device 10, 10" as shown Fig. 1, 3 instead of the device 10'. The dashed lines in Fig. 6 are merely for illustrative purposes and not to be understood as restricting the way of monitoring the subject 10 by the monitoring unit 36. In particular, the monitoring unit 36 may be configured to monitor the whole body of the subject 15 or part of it.

The one or more sensors may comprise any type of sensor capable of performing relevant physiological and/or behavioral measurements. In a preferable embodiment, the one or more sensors comprise at least one sensor for use under low light conditions. Such a sensor may be camera, such as an infrared (IR) camera which is capable of measuring night time behavior, so that the patient's health condition may be detected based on the measured night time behavior. An IR camera is advantageous for its capability of performing measurements contactlessly and for being easily installable. Unobstrusive monitoring is hence possible with high contextual stability. Further, IR cameras are known to capture a large context including a large area with a large number of subjects, a large number of different motions, a large context of the sleeping environment such as all people in a bed, their body activities, breathing and body position, etc. Finally, IR cameras are suitable for being used for long durations, in particular for several nights during readmission penalty period, so that readmission can be avoided. IR cameras may also be used for an even longer period of time. This enables comparing sensor data 14 originating from the long period, providing a basis for an improved parameter optimization and a more reliable health condition detection.

In another preferable embodiment, the one or more sensors of the monitoring unit 36 cooperate with and/or enable video analysis. This is advantageous since video analysis enables the monitoring of the body activity level of a patient, including a global full body actigraphy and/or a local body part motion. Video analysis also simplifies the classification of motions so that the physiological and/or behavioral features can more easily be extracted from the sensor data 14. The motion classifications preferably include turning, getting out of bed, moving legs, scratching, repetitive movements, abrupt and/or restless movements. The one or more sensors of the monitoring unit 36 may also detect breathing, body position, getting in or out of bed, the ON/OFF state of one or more light sources, etc. In a preferable embodiment, the afore-mentioned monitoring can be performed by the monitoring unit 36 in the common shared-bed situation.

In other preferable embodiments, the one or more sensors for the monitoring unit 36 comprise one or more microphones and/or accelerometers and or environmental sensors and or temperature sensors and or humidity sensors. The present invention is, however, not limited in the afore-mentioned types of sensors. Further embodiments include sleep monitoring devices such as Actiwatches, PSG devices.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for detecting a health condition of a subject (15), comprising:
- a data interface (12) for receiving sensor data (14) of said subject (15) and one or more disease classification parameters (16) for characterizing one or more diseases;
- an analysis unit (26) for extracting one or more physiological and/or behavioral features from said received sensor data (14); wherein the device is **characterized by** further comprising:
- a user interface (18) for receiving a user input (20) related to a disease activity perceived by the subject (15), wherein said disease activity comprises at least the occurrence of one or more symptoms;
- an optimizer unit (28) for optimizing said one or more disease classification parameters (16) based on a correlation analysis between said one or more physiological and/or behavioral features and said user input (20); and
- a detection unit (30) for detecting a health condition of said subject (15) by applying said one or more optimized disease classification parameters (16) to said one or more physiological and/or behavioral features.

2. The device according to claim 1, wherein said optimizer unit (28) is configured to correlate said user input (20) with said one or more physiological and/or behavioral features of a time different from a user input time, in particular earlier than said user input time.

3. The device according to claim 1, wherein said optimizer unit (28) is configured to optimize said one or more disease classification parameters (16) within a predefined time interval after said user interface (18) has received said user input (20).

4. The device according to claim 1, wherein said optimizer unit (28) is configured to define a combination from said one or more physiological and/or behavioral features.

5. The device according to claim 1, wherein said optimizer unit (28) is configured to derive at least one symptomatic characteristic from said one or more disease classification parameters (16), said symptomatic characteristic being configured to indicate a development and/or an activity state of said at least one disease.

6. The device according to claim 5, wherein said symptomatic characteristic is configured to indicate a disease activity decrease and/or a remission of at least one disease.

7. The device according to claim 5, wherein said symptomatic characteristic is configured to indicate a disease activity increase of at least one disease.

8. The device according to claim 5, wherein said symptomatic characteristic comprises a dynamic component variable in time.

9. The device according to claim 1, further being configured to derive at least one average disease classification parameter (16) from a plurality of individual disease classification parameters (16).

10. The device according to claim 1, wherein said user interface (18) is configured to compute a relative disease activity level based on a plurality of disease activity level inputs.

11. The device according to claim 1, wherein said optimizer unit (28) is configured to load said optimized one or more disease classification parameters (16) to a storage medium (32).

12. System (34) for detecting a health condition of a subject (15), comprising:
- a monitoring unit (36) comprising one or more sensors for measuring and/or monitoring sensor data (14) from said subject (15); and
- a device as claimed in claim 1 for detecting a health condition of said subject (15) by processing said measured and/or monitored sensor data (14).

13. The system (34) of claim 12, wherein at least one of the one or more sensors is a camera, preferably an infrared camera.

14. Computer program for detecting a health condition of a subject comprising program code means for causing a computer to carry out the following steps:
- receiving sensor data of said subject and one or more disease classification parameters for characterizing one or more diseases;
- extracting one or more physiological and/or behavioral features from said received sensor data;
- receiving a user input related to a disease activity perceived by the subject, wherein said disease activity comprises at least the occurrence of one or more symptoms;
- optimizing said one or more disease classification parameters based on a correlation analysis between said one or more physiological and/or behavioral features and said user input; and
- detecting a health condition of said subject by applying said one or more optimized disease classification parameters to said one or more physiological and/or behavioral features,
when said computer program is carried out on the computer.

## Patentansprüche

1. Vorrichtung zur Erkennung eines Gesundheitszustands einer Person (15), umfassend:
- eine Datenschnittstelle (12) zum Empfang von Sensordaten (14) der Person (15) und einen oder mehrere Krankheitsklassifizierungsparameter (16) zur Charakterisierung von einer oder mehreren Krankheiten;
- eine Analyseeinheit (26) zur Extraktion von einem oder mehreren physiologischen und oder Verhaltensmerkmalen aus den empfangenen Sensordaten (14); wobei die Vorrichtung **dadurch gekennzeichnet, dass** sie weiter Folgendes umfasst:
- eine Benutzerschnittstelle (18) zum Empfang einer Benutzereingabe (20), die mit einer Krankheitsaktivität verbunden ist, die von der Person (15) wahrgenommen wird, wobei die Krankheitsaktivität mindestens das Auftreten eines oder mehrerer Symptome umfasst;
- eine Optimierereinheit (28) zur Optimierung des einen oder der mehreren Krankheitsklassifizierungsparameter (16) basierend auf einer Korrelationsanalyse zwischen dem einen oder den mehreren physiologischen und/oder Verhaltensmerkmalen und der Benutzereingabe (20); und
- eine Nachweiseinheit (30) zum Nachweis eines Gesundheitszustands der Person (15) durch Anwendung des einen oder der mehreren optimierten Krankheitsklassifizierungsparameter (16) auf das eine oder die mehreren physiologischen und/oder Verhaltensmerkmale.

2. Vorrichtung nach Anspruch 1, wobei die Optimierereinheit (28) konfiguriert ist, um die Benutzereingabe (20) mit dem einen oder den mehreren physiologischen und/oder Verhaltensmerkmalen einer Zeit verschieden von einer Benutzereingabezeit, insbesondere früher als die Benutzereingabezeit, zu korrelieren.

3. Vorrichtung nach Anspruch 1, wobei die Optimierereinheit (28) konfiguriert ist, um den einen oder die mehreren Krankheitsklassifizierungsparameter (16) innerhalb eines vorbestimmten Zeitintervalls nachdem die Benutzerschnittstelle (18) die Benutzereingabe (20) empfangen hat, zu optimieren.

4. Vorrichtung nach Anspruch 1, wobei die Optimierereinheit (28) konfiguriert ist, um eine Kombination von der einen oder den mehreren physiologischen und/oder Verhaltensmerkmalen zu definieren.

5. Vorrichtung nach Anspruch 1, wobei die Optimierereinheit (28) konfiguriert ist, um das mindestens eine symptomatische Kennzeichen von dem einen oder den mehreren Krankheitsklassifizierungsparametern (16) abzuleiten, wobei das symptomatische Kennzeichen konfiguriert ist, um eine Entwicklung und/oder einen Aktivitätszustand der mindestens einen Krankheit anzuzeigen.

6. Vorrichtung nach Anspruch 5, wobei das symptomatische Kennzeichen konfiguriert ist, um eine Abnahme der Krankheitsaktivität und/oder ein Nachlassen von mindestens einer Krankheit anzuzeigen.

7. Vorrichtung nach Anspruch 5, wobei das symptomatische Kennzeichen konfiguriert ist, um eine Zunahme der Krankheitsaktivität von mindestens einer Krankheit anzuzeigen.

8. Vorrichtung nach Anspruch 5, wobei das symptomatische Kennzeichen eine zeitvariable dynamische Komponente umfasst.

9. Vorrichtung nach Anspruch 1, die weiter konfiguriert ist, um mindestens einen durchschnittlichen Krankheitsklassifizierungsparameter (16) aus einer Vielzahl von individuellen Krankheitsklassifizierungsparametern (16) abzuleiten.

10. Vorrichtung nach Anspruch 1, wobei die Benutzerschnittstelle (18) konfiguriert ist, um ein relatives Krankheitsaktivitätsniveau basierend auf einer Vielzahl von Krankheitsaktivitätsniveau-Eingaben zu berechnen.

11. Vorrichtung nach Anspruch 1, wobei die Optimierereinheit (28) konfiguriert ist, um den einen oder die mehreren optimierten Krankheitsklassifizierungsparameter (16) in ein Speichermedium (32) zu laden.

12. System (34) zur Erkennung eines Gesundheitszustands einer Person (15), umfassend:
- eine Überwachungseinheit (36), umfassend einen oder mehrere Sensoren, zum Messen und/oder Überwachen von Sensordaten (14) von der Person (15); und
- eine Vorrichtung nach Anspruch 1 zum Nachweis eines Gesundheitszustands der Person (15) durch Verarbeiten des gemessenen und/oder überwachten Sensordaten (14).

13. System (34) nach Anspruch 12, wobei mindestens einer des einen oder der mehreren Sensoren eine Kamera ist, vorzugsweise eine Infrarotkamera.

14. Computerprogramm zum Nachweis eines Gesundheitszustands einer Person, umfassend Programmcodemittel zum Verursachen, dass ein Computer die folgenden Schritte durchführt:
- Empfangen von Sensordaten der Person und eines oder mehrerer Krankheitsklassifizierungsparameter zur Charakterisierung von einer oder mehreren Krankheiten;
- Extrahieren von einem oder mehreren physiologischen und/oder Verhaltensmerkmalen aus den erhaltenen Sensordaten;
- Empfangen einer Benutzereingabe, die mit einer Krankheitsaktivität verbunden ist, die von der Person wahrgenommen wird, wobei die Krankheitsaktivität mindestens das Auftreten eines oder mehrerer Symptome umfasst;
- Optimieren des einen oder der mehreren Krankheitsklassifizierungsparameter basierend auf einer Korrelationsanalyse zwischen dem einen oder den mehreren physiologischen und/oder Verhaltensmerkmalen und der Benutzereingabe; und
- Nachweisen eines Krankheitszustands der Person durch Anwenden des einen oder der mehreren optimierten Krankheitsklassifizierungsparameter auf das eine oder die mehreren physiologischen und/oder Verhaltensmerkmale,
wenn das Computerprogramm auf dem Computer durchgeführt wird.

## Revendications

1. Dispositif permettant de détecter un état de santé d'un sujet (15), comprenant :
- une interface de données (12) permettant de recevoir des données de capteur (14) dudit sujet (15) et un ou plusieurs paramètres de classification de maladies (16) permettant de caractériser une ou plusieurs maladies ;
- une unité d'analyse (26) permettant d'extraire une ou plusieurs particularités physiologiques et/ou comportementales à partir desdites données de capteur (14) reçues ; dans lequel le dispositif est **caractérisé en ce qu'**il comprend en outre :
- une interface utilisateur (18) permettant de recevoir une entrée utilisateur (20) liée à une activité de maladie perçue par le sujet (15), dans lequel ladite activité de maladie comprend au moins l'apparition d'un ou plusieurs symptômes ;
- une unité d'optimisation (28) permettant d'optimiser lesdits un ou plusieurs paramètres de classification de maladies (16) sur la base d'une analyse de corrélation entre lesdites une ou plusieurs particularités physiologiques et/ou comportementales et ladite entrée utilisateur (20) ; et
- une unité de détection (30) permettant de détecter un état de santé dudit sujet (15) en appliquant lesdits un ou plusieurs paramètres de classification de maladies (16) optimisés auxdites une ou plusieurs particularités physiologiques et/ou comportementales.

2. Dispositif selon la revendication 1, dans lequel ladite unité d'optimisation (28) est configurée pour mettre en corrélation ladite entrée utilisateur (20) avec lesdites une ou plusieurs particularités physiologiques et/ou comportementales d'un temps différent d'un temps d'entrée utilisateur, en particulier plus tôt que ledit temps d'entrée utilisateur.

3. Dispositif selon la revendication 1, dans lequel ladite unité d'optimisation (28) est configurée pour optimiser lesdits un ou plusieurs paramètres de classification de maladies (16) dans un intervalle de temps prédéfini après que ladite interface utilisateur (18) a reçu ladite entrée utilisateur (20).

4. Dispositif selon la revendication 1, dans lequel ladite unité d'optimisation (28) est configurée pour définir une combinaison à partir desdites une ou plusieurs particularités physiologiques et/ou comportementales.

5. Dispositif selon la revendication 1, dans lequel ladite unité d'optimisation (28) est configurée pour déduire au moins une caractéristique symptomatique à partir desdits un ou plusieurs paramètres de classification de maladies (16), ladite caractéristique symptomatique étant configurée pour indiquer un développement et/ou un état d'activité de ladite au moins une maladie.

6. Dispositif selon la revendication 5, dans lequel ladite caractéristique symptomatique est configurée pour indiquer une diminution d'activité de maladie et/ou une rémission d'au moins une maladie.

7. Dispositif selon la revendication 5, dans lequel ladite caractéristique symptomatique est configurée pour indiquer une augmentation d'activité de maladie d'au moins une maladie.

8. Dispositif selon la revendication 5, dans lequel ladite caractéristique symptomatique comprend une composante dynamique variable dans le temps.

9. Dispositif selon la revendication 1, qui est configuré en outre pour déduire au moins un paramètre de classification de maladies (16) moyen à partir d'une pluralité de paramètres de classification de maladies (16) individuels.

10. Dispositif selon la revendication 1, dans lequel ladite interface utilisateur (18) est configurée pour calculer un niveau d'activité de maladie relatif sur la base d'une pluralité d'entrées de niveau d'activité de maladie.

11. Dispositif selon la revendication 1, dans lequel ladite unité d'optimisation (28) est configurée pour charger lesdits un ou plusieurs paramètres de classification de maladies (16) optimisés dans un support de stockage (32).

12. Système (34) permettant de détecter un état de santé d'un sujet (15), comprenant :
- une unité de surveillance (36) comprenant un ou plusieurs capteurs permettant de mesurer et/ou de surveiller des données de capteur (14) provenant dudit sujet (15) ; et
- un dispositif selon la revendication 1 permettant de détecter un état de santé dudit sujet (15) en traitant lesdites données de capteur (14) mesurées et/ou surveillées.

13. Système (34) de la revendication 12, dans lequel au moins un des un ou plusieurs capteurs est une caméra, de préférence une caméra infrarouge.

14. Programme d'ordinateur permettant de détecter un état de santé d'un sujet comprenant un moyen de code de programme permettant d'amener un ordinateur à mettre en oeuvre les étapes suivantes :
- la réception de données de capteur dudit sujet et d'un ou plusieurs paramètres de classification de maladies permettant de caractériser une ou plusieurs maladies ;
- l'extraction d'une ou plusieurs particularités physiologiques et/ou comportementales à partir desdites données de capteur reçues ;
- la réception d'une entrée utilisateur liée à une activité de maladie perçue par le sujet, dans lequel ladite activité de maladie comprend au moins l'apparition d'un ou plusieurs symptômes ;
- l'optimisation desdits un ou plusieurs paramètres de classification de maladies sur la base d'une analyse de corrélation entre lesdites une ou plusieurs particularités physiologiques et/ou comportementales et ladite entrée utilisateur ; et
- la détection d'un état de santé dudit sujet en appliquant lesdits un ou plusieurs paramètres de classification de maladies optimisés auxdites une ou plusieurs particularités physiologiques et/ou comportementales,
lorsque ledit programme d'ordinateur est mis en oeuvre sur l'ordinateur.
